# EUROPÄISCHE PATENTSCHRIFT

(11) **EP 2 529 199 B1**
(45) Veröffentlichungstag und Bekanntmachung des Hinweises auf die Patenterteilung: **10.06.2015**
(21) Anmeldenummer: 11700860.7
(22) Anmeldetag: 27.01.2011
(51) Int. Cl.: G01N 21/31, G01N 21/84

(54) **MESSSYSTEM UND MESSVERFAHREN ZUR BLUTZUCKERBESTIMMUNG**
MEASURING SYSTEM AND MEASURING METHOD FOR DETERMINING BLOOD GLUCOSE
SYSTÈME ET PROCÉDÉ DE MESURE DESTINÉ À LA DÉTERMINATION DE LA GLYCÉMIE

(30) Priorität: 28.01.2010 EP 10152018
(43) Veröffentlichungstag der Anmeldung: 05.12.2012
(73) Patentinhaber: Roche Diagnostics GmbH, 68305 Mannheim (DE); F.Hoffmann-La Roche AG, 4070 Basel (CH)
(72) Erfinder: HOENES, Joachim, 64673 Zwingenberg (DE); WEHOWSKI, Frederic, 68766 Hockenheim (DE); MISCHLER, Reinhold, 67063 Ludwigshafen (DE); HARTTIG, Herbert, 67434 Neustadt (DE)
(74) Vertreter: Pfiz, Thomas
(86) Internationale Anmeldenummer: PCT/EP2011/051168
(87) Internationale Veröffentlichungsnummer: WO 2011/092259

(56) Entgegenhaltungen:
- WO-A1-2010/038024
- WO-A2-2006/037985
- US-A- 5 089 229
- US-A1- 2002 030 292
- US-A1- 2003 058 450
- US-A1- 2008 291 455
- OKAMURA Y ET AL: "Characteristics of modulated white LED and their application to electrically controlled spectroscopy", PROCEEDINGS OF THE INTERNATIONAL SOCIETY FOR OPTICAL ENGINEERING (SPIE), SPIE, USA, Bd. 4922, 1. Januar 2002 (2002-01-01), Seiten 43-50, XP002558253, ISSN: 0277-786X

## Beschreibung

Die Erfindung betrifft ein Messsystem zur Blutzuckerbestimmung mit einer photometrischen Messeinheit, die eine Lichtquelle und einen Detektor umfasst, und mit einem mit einer Körperflüssigkeit beaufschlagbaren und für einen optischen Nachweis eines Analyten in einen Strahlengang zwischen der Lichtquelle und dem Detektor bringbaren analytischen Testelement. Die Erfindung betrifft weiter ein entsprechendes Messverfahren.

In der Praxis für Blutzuckerbestimmungen bekannte Systeme dieser Art basieren auf irreversibel reagierenden trägergebundenen Testelementen in Form von Teststreifen oder Testbändern. Diese sollen auch dem Laien durch Verarbeitung in automatischen kompakten Handgeräten die Messung von Blutglucose außerhalb einer Laborumgebung mit hinreichender Genauigkeit ermöglichen, wie sie die medizinische Behandlung von Diabetes erfordert. Der Messablauf sieht vor, dass nach Aufbringen einer Blutprobe die Analytkonzentration durch zweckmäßig wiederholte photometrische Messungen ermittelt wird. Dabei ist es wichtig, dass Veränderungen in den Randbedingungen der Messung unabhängig von der eigentlichen Analyterfassung erkannt werden.

US Patent Nr. 5,089,229 offenbart ein für die Glukosebestimmung geeignetes Analysegerät. Das Analysegerät verfügt zur photometrischen Untersuchung einer auf einen Testträger aufgetragenen Probe über mehrere Lichtquellen, die Licht unterschiedlicher Wellenlängen emittieren.

Ausgehend hiervon liegt der Erfindung die Aufgabe zugrunde, die im Stand der Technik bekannten Vorrichtungen und Verfahren und insbesondere die Qualität und Genauigkeit des Messprozesses weiter zu verbessern, wobei mit begrenztem Aufwand ein effizientes und kompaktes System geschaffen werden soll.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Dementsprechend wird im Hinblick auf ein Messsystem vorgeschlagen, dass die Lichtquelle einen in einem ersten Wellenlängenbereich zur Aussendung von pulsierendem Wechsellicht angesteuerten ersten Strahler und einen in einem zweiten Wellenlängenbereich zur Emission von Fluoreszenzlicht angeregten zweiten Strahler aufweist. Damit lässt sich eine kompakte und effiziente Mehrwellenlängenlichtquelle realisieren, die regelbar Strahlung in verschiedenen Wellenlängenbereichen abgibt und eine selektive Signalauswertung gestattet. Somit ist es auch möglich, zeitgleich eine Analyt- und Kontrollmessung durchzuführen, um so die erforderliche Messqualität sicherstellen zu können.

Für eine selektive Signalerfassung ist es vorteilhaft wenn das Wechsellicht eine Impulsdauer aufweist und das Fluoreszenzlicht mit einer Fluoreszenzlebensdauer abklingt, und wenn die Fluoreszenzlebensdauer um ein Vielfaches größer als die Impulsdauer ist. Damit können unterschiedliche Lebensdauern bzw. Abklingzeiten in den verschiedenen Wellenlängenbereichen zur elektronischen Selektion anstelle einer aufwändigen Wellenlängenselektion mit Filtern oder ähnlichem genutzt werden.

Eine baulich vorteilhafte Ausführung sieht vor, dass der erste Strahler durch eine insbesondere im UV-Bereich emittierende Leuchtdiode gebildet ist. Vorteilhaft ist es auch, wenn der zweite Strahler durch einen Leuchtstoff gebildet ist, welcher durch den gepulsten ersten Strahler zur Abgabe von insbesondere sichtbarem Fluoreszenzlicht optisch angeregt wird. Leuchtdioden weisen hohe Leuchtdichten auf und können daher gut für intensive gebündelte Lichtstrahlen eingesetzt werden. Durch die Kombination mit einem Leuchtstoff kann auf mehrere Einzel-LEDs, die an verschiedenen Stellen sitzen und nur aufwändig zu einem homogenen Lichtstrahl gebündelt werden können, verzichtet werden.

Besonders bevorzugt sind beide Strahler über einen einheitlichen optischen Übertragungsweg bzw. Strahlengang gemeinsam auf eine Messfläche des Testelements ausgerichtet. Eine weitere Verbesserung vor allem hinsichtlich des erforderlichen Bauraums ergibt sich dadurch, dass der zweite Strahler als fluoreszierende Leuchtstoffschicht auf eine Abstrahlfläche des ersten Strahlers aufgebracht ist, so dass sich beide Lichtanteile durch ein einziges Bauteil erzeugen lassen.

Vorteilhafterweise weist der Detektor einen Photoempfänger zur gemeinsamen Erfassung des Wechsel- und Fluoreszenzlichts und zwei Verstärkerkanäle zur wellenlängenselektiven Messwertbestimmung auf.

Zur zeitaufgelösten separaten Erfassung eines durch das Wechsellicht erzeugten Wechsellicht-Signalanteils ist es vorteilhaft, wenn der Detektor einen mit der Impulsfrequenz des Wechsellichts modulierbaren Lock-in-Verstärker aufweist.

Für die Signalverarbeitung ist es von Vorteil, wenn der Detektor einen insbesondere als Integrator arbeitenden Verstärker zur Erfassung eines durch das Wechsellicht und Fluoreszenzlicht erzeugten Summensignals und einen Signalprozessor zur Bestimmung von Signalanteilen des Wechsellichts und/oder Fluoreszenzlichts, insbesondere zur Subtraktion des Wechsellicht-Signalanteils von dem Summensignal umfasst.

Für patientennahe Messungen vor Ort ist es günstig, wenn die Messeinheit in einem Handgerät integriert ist und das Testelement als Verbrauchsmittel für einen Einmaleinsatz in dem Handgerät ausgelegt ist.

Das Testelement ist für eine reflexionsphotometrische Messung als Reflektor im Strahlengang zwischen Lichtquelle und Detektor angeordnet und ermöglicht bevorzugt durch eine mit der Probe kontaktierte Reagenzschicht den photometrischen Nachweis eines Analyten in der Probe. Hierfür weist das Testelement vorteilhafterweise einen Aufnahmebereich zum Auftragen der Probe auf, während eine ggf. von dem Aufnahmebereich entfernte oder rückseitig abgewandte Messzone des Testelements die Strahlung von den Strahlern der Lichtquelle empfängt und diese in Richtung des Detektors bevorzugt streuend reflektiert. Durch die Probe bzw. einen darin befindlichen Analyten ändern sich optische Eigenschaften des Testelements, die zu einem entsprechend veränderten Messsignal führen.

Eine besonders vorteilhafte Ausführung sieht vor, dass das Testelement über eine Optikeinheit, insbesondere einen Lichtleiter an die Lichtquelle angekoppelt ist, und dass die optische Ankopplung durch eine gesonderte Signalauswertung in einem der Wellenlängenbereiche kontrolliert wird. Für die Einkoppelung in dünne Lichtleiter ist eine möglichst kleine Lichtquelle nötig, um hohe Einkoppeleffizienz zu erreichen. Hierbei ist anzumerken, dass sich mehrere gesonderte LEDs nur unter hohen Verlusten einkoppeln lassen.

In verfahrensmäßiger Hinsicht wird die eingangs genannte Aufgabe dadurch gelöst, dass ein erster Strahler der Lichtquelle in einem ersten Wellenlängenbereich zur Aussendung von pulsierendem Wechsellicht angesteuert wird, und dass ein zweiter Strahler in einem zweiten Wellenlängenbereich zur Emission von dem Wechsellicht überlagertem Fluoreszenzlicht angeregt wird.

Dabei kann durch eine zeitaufgelöste Signalerfassung vorzugsweise mittels eines Lock-in-Verstärkers ein dem Wechsellicht zugeordneter Signalanteil erfasst werden. Ein weiterer Vorteil ergibt sich dadurch, dass in einem Wellenlängenbereich ein Messwert für einen Analyten in der Probe und in dem anderen Wellenlängenbereich ein Kontrollwert für die optische Ankopplung des Testelements an die Messeinheit erfasst wird.

Im Folgenden wird die Erfindung anhand der in der Zeichnung schematisch dargestellten Ausführungsbeispiele näher erläutert. Es zeigen
- Fig. 1: ein Blockschaltbild eines Messsystems zur Blutzuckermessung mit einem Mehrwellenlängen-Photometer;
- Fig. 2: einen Zeitverlauf der Strahlungsintensität eines Impulsstrahlers und eines Fluoreszenzstrahlers des Photometers nach Fig. 1;
- Fig. 3: die Strahlungsintensität auf einer logarithmischen Zeitskala für ein weiteres Ausführungsbeispiel einer Mehrwellenlängenmessung.

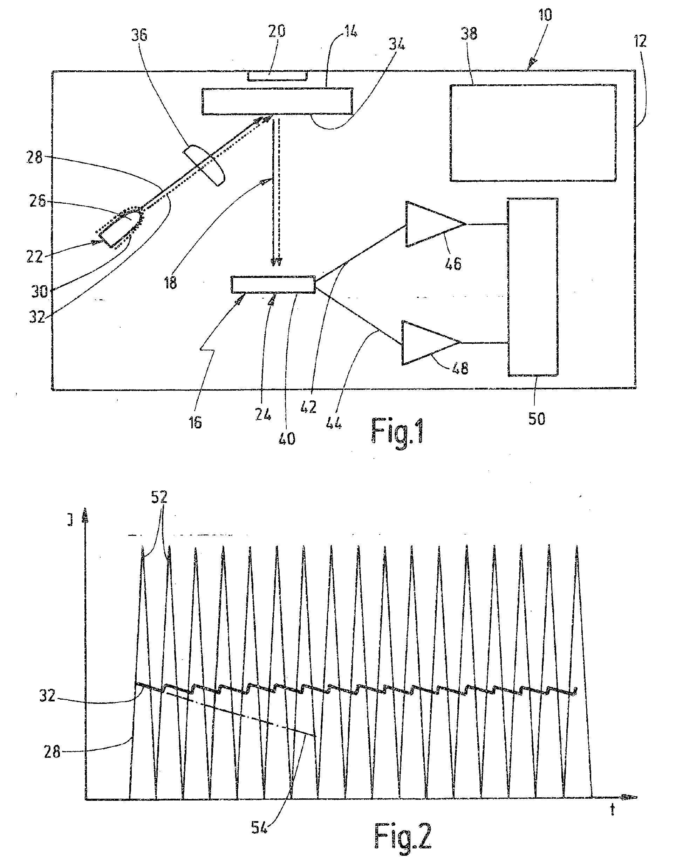

Das in Fig. 1 dargestellte Messsystem 10 umfasst ein tragbares Testgerät 12 und darin einsetzbare Testelemente 14 für jeweils einen Einmaltest an einer Probe, speziell zur Glukosebestimmung in einer Blutprobe. Zu diesem Zweck weist das Testgerät 12 eine photometrische Messeinheit 16 auf, in deren Strahlengang 18 ein disposibles Testelement 14 beispielsweise in Form eines Teststreifens oder Testbands einbringbar ist, wobei über eine Aufnahme 20 für ein Körperteil ein direkter Probenauftrag auf das Testelement 14 möglich ist. Das Testelement 14 ist mit einer trockenchemischen Reagenzschicht versehen, die auf den Analyten Glucose durch einen photometrisch messbaren Farbumschlag reagiert. Die Reagenzschicht kann als abgegrenztes Testfeld auf einem transparenten Träger aufgebracht sein.

Die photometrische Messeinheit 16 umfasst eine Mehrwellenlängenlichtquelle 22 und einen zweikanaligen Detektor 24. Ein erster, gepulster Strahler 26 der Lichtquelle 22 ist zur Aussendung von pulsierendem Wechsellicht 28 betrieben, während ein zweiter, fluoreszierender Strahler 30 durch das Licht des ersten Strahlers 26 zur Emission von längerwelligem Fluoreszenzlicht 32 angeregt ist. Der erste Strahler 26 ist zweckmäßig durch eine im UV-Bereich emittierende Leuchtdiode und der zweite Strahler durch eine im sichtbaren Wellenlängenbereich fluoreszierende Leuchtstoffschicht auf der Leuchtdiode gebildet. Auf diese Weise sind beide Strahler 26, 28 als einheitliches Bauteil bestrombar und gemeinsam auf eine Messfläche 34 des Testelements 14 ausgerichtet. Zur Lichtbündelung auf einen möglichst kleinen Messfleck kann eine Sammellinse 36 und/oder ein Lichtleiter in dem gemeinsamen optischen Übertragungsweg der beiden Strahler vorgesehen sein.

Um Zustandsänderungen der optischen Ankopplung des Testelements 14 zu erkennen und das optische Übertragungsverhalten zu überprüfen, kann mittels des Fluoreszenzlichts ein Kontrollwert bestimmt werden, während das auf den Analyten ansprechende UV-Licht die Bestimmung eines Messwerts ermöglicht, der auf dem Display 38 in Form einer digitalen Konzentrationsangabe für einen Benutzer anzeigbar ist.

Die Kontrollwertbestimmung ermöglicht es, eine unbeabsichtigte Beeinflussung der Messbedingungen durch eine geräteseitige Aktuation oder benutzerseitige Eingriffe im Zuge der Bereitstellung des Testelements zu erkennen. Beispielsweise kann der Probenauftrag durch Andrücken eines Körperteils auf das Testelement 14 zu einer ungewollten Verformung oder Verschiebung führen. Hinzu kommt, dass die Erfassung der Messwerte zum Startzeitpunkt der Probenapplikation unsicher bzw. ungenau ist, weil die Testfeldbenetzung nicht schlagartig homogen erfolgt und die optischen Eigenschaften sich ändern. Um hier Abhilfe zu schaffen, kann durch eine Zweiwellenlängenmessung die erforderliche Messgenauigkeit und Robustheit gegen Störungen mit geringem apparativem Aufwand sichergestellt werden.

Zu diesem Zweck weist der Detektor 24 einen in beiden Messwellenlängenbereichen empfindlichen Photoempfänger 40 und zwei daran angeschlossene Verstärkerstufen bzw. -kanäle 42, 44 zur wellenlängenselektiven Messwertbestimmung auf. In der ersten Verstärkerstufe 42 ist ein Lock-in-Verstärker 46 zur zeitaufgelösten Erfassung eines Wechsellicht-Signalanteils angeordnet. In der zweiten Verstärkerstufe befindet sich ein integrierender Verstärker 48 zur integralen Erfassung eines durch das Wechsellicht und das Fluoreszenzlicht erzeugten Summensignals. Ein nachgeordneter Signalprozessor 50 ermöglicht die Subtraktion des Wechsellicht-Signalanteils von dem Summensignal und somit die separate Bestimmung des Fluoreszenzlicht- bzw. Gleichlicht-Signalanteils.

Wie aus Fig. 2 ersichtlich, kann der erste Strahler 26 durch schnelle Stromimpulse beispielsweise im Mikrosekundentakt zur Aussendung von pulsierendem Wechsellicht angesteuert werden. Das UV-Licht 28 wird durch die Stromimpulse nahezu verzögerungsfrei erzeugt, so dass sich einzeln auflösbare Lichtimpulse 52 mit einer Impulsdauer von 1 µs ergeben. Das dadurch angeregte Fluoreszenzlicht des zweiten Strahlers kann diesem Rhythmus jedoch nicht folgen, da es mit einer wesentlich längeren Zeitkonstante abklingt. In dem gezeigten Beispiel ist durch die strichpunktierte Kurve 54 der Signalabfall für einen Fluorophor als Leuchtschicht 30 mit einer Fluoreszenzlebensdauer von z.B. 20 µs verdeutlicht. Beispielhaft zu nennen ist hier der im Handel unter der Markenbezeichnung Lumilux CD163 von der Firma Honeywell erhältliche Fluorophor, der im grünen Wellenlängenbereich bei max. 517 nm fluoresziert. Aufgrund der schnellen Impulsfolge und der relativ langen Fluoreszenzlebensdauer wird der Signalabfall entsprechend verkürzt, so dass ein Gleichlichtanteil mit nur geringer Restwelligkeit beobachtbar ist. Somit ergibt sich also ein schneller Wechsel des kurzwelligen Lichts 28 überlagert durch fast konstantes Gleichlicht des längerwelligen Fluoreszenzlichts 32. Da bei der Detektion zwischen Gleichlichtanteil und pulsierendem Anteil des Lichts differenziert werden kann, ist es möglich, die von den beiden Strahlern 26, 28 abgegebenen verschiedenen Lichtanteile ohne Einsatz von Wellenlängenfiltern o.ä. getrennt zu messen.

Die Messung des Wechsellichtanteils ist mit dem Lock-in-Verstärker 46 auf besonders einfache Weise möglich. Hierbei wird ein Referenzsignal mit der Frequenz des modulierten UV-Lichts erzeugt und durch einen Phasenschieber in seiner Phasenlage angepasst. Alternativ zu einem Phasenschieber kann auch ein so genannter Double-lock-Verstärker eingesetzt werden, der durch eine Doppelmessung bei den Phasenlagen 0 und 90 Grad das Wechsellicht phasenunabhängig erfassen kann. Durch einen Multiplizierer wird dann das eigentliche Messsignal mit dem Referenzsignal multipliziert, so dass nur der Wechsellichtanteil ein endliches Ausgangssignal liefert und der Hauptteil des grünen Gleichlichts nicht miterfasst wird.

Eine Signalintegration mittels des Verstärkers 48 über längere Zeit ergibt die Summe von UV-Licht 28 und grünem Licht 32. Sodann kann ein einfacher Algorithmus in dem Signalprozessor 50 den Wechsellichtanteil aus dem Summensignal subtrahieren, um so einen separaten Messwert für den Gleichlichtanteil zu erhalten.

Grundsätzlich ist es nicht erforderlich, Wechsellicht und Gleichlicht mit sehr stark unterschiedlichen Frequenzen völlig getrennt zu erfassen. Auch bei näher zusammen liegenden Frequenzen ergeben sich zwei unterschiedliche Signale mit unterschiedlichen Anteilen der beiden Wellenlängen, die sich durch Lösen eines Gleichungssystems (zwei Gleichungen mit zwei Unbekannten) ermitteln lassen. Der Integrator kann durch einen Verstärker mit geeigneter anderer Frequenz ersetzt werden, und der Prozessor muss nicht auf eine einfache Subtraktion beschränkt sein.

Wie aus der folgenden Tabelle schematisch hervorgeht, lässt sich das erläuterte Mehrwellenlängen-Messprinzip mit geeigneten Fluorophoren abgestufter Abklingzeiten auch auf mehr als zwei Wellenlängen bzw. Lichtfarben ausweiten. Als Grenzfrequenz für das Erreichen einer hohen Pulsamplitude ist hierbei die Frequenz angegeben, die der zehnfachen Abklingzeit entspricht.

| Wellenlänge | Abklingzeit | Grenzfrequenz |
|---|---|---|
| Ultraviolett | 10ns (LED) | 10 MHz |
| Blau | 1µs (LUMILUX Blau) | 100 kHz |
| Grün | 100µs (LUMILUX Grün) | 1 kHz |

Das Wechsellicht von 10 MHz enthält somit nur die UV-Intensität, das Wechsellicht von 100 kHz die Summe von UV und Blau, während bei Frequenzen von 1 kHz und kleiner die gesamte Lichtintensität erfasst wird.

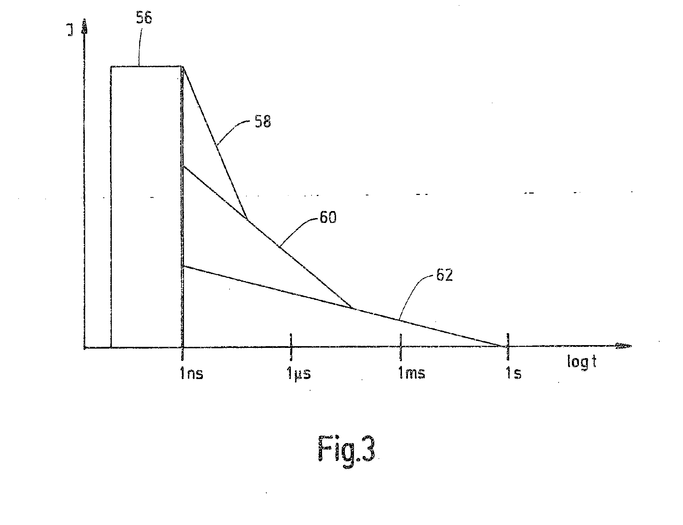

Die Messung lässt sich auch gemäß Fig. 3 durchführen, indem mittels eines Stromimpulses ein rechteckförmiger Lichtimpuls 56 erzeugt und dessen Abklingen analysiert wird. In den einzelnen Zeitbereichen der logarithmischen Zeitskala lässt sich jeweils das Abklingen eines Wellenlängenbereiches 58, 60, 62 separat erfassen. Dieses Verfahren entspricht dem Messverfahren, das bei der Bestimmung von Fluoreszenzlebensdauern an sich bekannt ist. Die exponentielle Abklingkurve wird dabei mit einer, zwei oder drei Fluoreszenzlebensdauern und dazugehörigen Intensitätsanteilen als Fitparameter angepasst. In der vorliegenden Anwendung sind die charakteristischen Lebensdauern in den drei Wellenlängenbereichen 58, 60, 62 von vornherein bekannt. Daher kann ein Fit mit nur drei Parametern, nämlich den drei Intensitätsanteilen in den jeweiligen Wellenlängenbereichen, durchgeführt werden. Die UV-Strahlung folgt dem Abklingen der LED, die Abklingkurven der Fluorophore folgen den Fluoreszenzlebensdauern, die zweckmäßigerweise hinreichend unterschiedlich gewählt sind, beispielsweise um einen Faktor von mindestens 3. Hierbei ist abzuwägen zwischen der Möglichkeit zur schärferen Wellenlängendifferenzierung durch größere Lebensdauerunterschiede und einer verlängerten Messzeit bei verlängerter Abklingdauer.

## Patentansprüche

1. Messsystem zur Blutzuckerbestimmung mit einer photometrischen Messeinheit (16), die eine Lichtquelle (22) und einen Detektor (24) umfasst, und einem mit einer Körperflüssigkeit beaufschlagbaren und für einen optischen Nachweis eines Analyten in einen Strahlengang (18) zwischen der Lichtquelle (22) und dem Detektor (24) bringbaren analytischen Testelement (14), **dadurch gekennzeichnet, dass** die Lichtquelle (22) einen zur Aussendung von pulsierendem Wechsellicht (28) in einem ersten Wellenlängenbereich angesteuerten ersten Strahler (26) und einen durch einen Leuchtstoff gebildeten, durch den gepulsten ersten Strahler (26) zur Emission von Fluoreszenzlicht (32) in einem zweiten Wellenlängenbereich angeregten zweiten Strahler (30) aufweist, wobei beide Strahler gemeinsam auf eine Messfläche (34) des Testelements (14) ausgerichtet sind.

2. Messsystem nach Anspruch 1, **dadurch gekennzeichnet, dass** das Wechsellicht (28) eine Impulsdauer aufweist und das Fluoreszenzlicht (32) mit einer Fluoreszenzlebensdauer abklingt, und dass die Fluoreszenzlebensdauer um ein Vielfaches größer als die Impulsdauer ist.

3. Messsystem nach Anspruch 1 oder 2, **dadurch gekennzeichnet, dass** der erste Strahler (26) durch eine insbesondere im UV-Bereich emittierende Leuchtdiode gebildet ist.

4. Messsystem nach einem der Ansprüche 1 bis 3, **dadurch gekennzeichnet, dass** der zweite Strahler (30) zur Abgabe von sichtbarem Fluoreszenzlicht (32) optisch anregbar ist.

5. Messsystem nach einem der Ansprüche 1 bis 4, **dadurch gekennzeichnet, dass** der zweite Strahler (30) als fluoreszierende Leuchtstoffschicht auf eine Abstrahlfläche des ersten Strahlers (26) aufgebracht ist.

6. Messsystem nach einem der Ansprüche 1 bis 5, **dadurch gekennzeichnet, dass** der Detektor (24) einen Photoempfänger (40) zur gemeinsamen Erfassung des Wechsel- und Fluoreszenzlichts (28,32) aufweist.

7. Messsystem nach einem der Ansprüche 1 bis 6, **dadurch gekennzeichnet, dass** der Detektor (24) zwei Verstärkerkanäle (42,44) zur wellenlängenselektiven Messwertbestimmung aufweist.

8. Messsystem nach einem der Ansprüche 1 bis 7, **dadurch gekennzeichnet, dass** der Detektor (24) einen mit der Impulsfrequenz des Wechsellichts (28) modulierbaren Lock-in-Verstärker (46) zur Erfassung eines durch das Wechsellicht (28) erzeugten Wechsellicht-Signalanteils aufweist.

9. Messsystem nach einem der Ansprüche 1 bis 8, **dadurch gekennzeichnet, dass** der Detektor (24) einen insbesondere als Integrator (48) arbeitenden Verstärker zur Erfassung eines durch das Wechsellicht (28) und Fluoreszenzlicht (32) erzeugten Summensignals umfasst.

10. Messsystem nach einem der Ansprüche 1 bis 9, **dadurch gekennzeichnet, dass** der Detektor (24) einen Signalprozessor (50) zur Bestimmung von Signalanteilen des Wechsellichts und/oder Fluoreszenzlichts, insbesondere zur Subtraktion des Wechsellicht-Signalanteils von dem Summensignal aufweist.

11. Messsystem nach einem der Ansprüche 1 bis 10, **dadurch gekennzeichnet, dass** die Messeinheit (16) in einem Handgerät (12) integriert ist und das Testelement (14) als Verbrauchsmittel für einen Einmaleinsatz in dem Handgerät (12) ausgelegt ist.

12. Messsystem nach einem der Ansprüche 1 bis 11, **dadurch gekennzeichnet, dass** das Testelement (14) über eine Optikeinheit (36), insbesondere Linse oder einen Lichtleiter an die Lichtquelle (22) ankoppelbar ist, und dass die optische Ankopplung durch eine gesonderte Signalauswertung in einem der Wellenlängenbereiche kontrollierbar ist.

13. Messverfahren zur Blutzuckerbestimmung bei welchem eine Körperflüssigkeit auf ein als Verbrauchsmittel einsetzbares analytisches Testelement (14) aufgebracht wird und das Testelement (14) mittels einer eine Lichtquelle (22) und einen Detektor (24) umfassenden photometrischen Messeinheit (16) optisch abgetastet wird, **dadurch gekennzeichnet, dass** ein erster Strahler (26) der Lichtquelle (22) zur Aussendung von pulsierendem Wechsellicht (28) in einem ersten Wellenlängenbereich angesteuert wird, und dass durch den gepulsten ersten Strahler (26) ein durch einen Leuchtstoff gebildeter zweiter Strahler (30) zur Emission von dem Wechsellicht (28) überlagertem Fluoreszenzlicht (32) in einem zweiten Wellenlängenbereich angeregt wird, wobei beide Strahler gemeinsam auf eine Messfläche (34) des Testelements (14) ausgerichtet werden.

14. Messverfahren nach Anspruch 13, **dadurch gekennzeichnet, dass** durch eine zeitaufgelöste Signalerfassung vorzugsweise mittels eines Lock-in-Verstärkers (46) ein dem Wechsellicht (28) zugeordneter Signalanteil ermittelt wird.

15. Messverfahren nach Anspruch 13 oder 14, **dadurch gekennzeichnet, dass** in einem Wellenlängenbereich ein Messwert für einen Analyten in der Probe und in dem anderen Wellenlängenbereich ein Kontrollwert für die optische Ankopplung des Testelements (14) an die Messeinheit (16) erfasst wird.

## Claims

1. A measuring system for blood glucose determination having a photometric measuring unit (16) comprising a light source (22) and a detector (24), and an analytical test element (14) to which a body fluid can be applied and which can be placed in a beam path (18) between the light source (22) and the detector (24) for optical detection of an analyte, **characterized in that** the light source (22) includes a first emitter (26) that is actuated in a first wavelength range for emitting pulsed alternating light (28) and a second emitter (30) that is formed by a fluorescent substance and is excited by the pulsed first emitter (26) to emit fluorescent light (32) in a second wavelength range, wherein both emitters are jointly oriented towards a measuring surface (34) of the test element (14).

2. The measuring system according to Claim 1, **characterized in that** the alternating light (28) has a pulse duration and the fluorescent light (32) decays with a fluorescent life cycle, and that the fluorescent life cycle is multiple times greater than the pulse duration.

3. The measuring system according to Claim 1 or 2, **characterized in that** the first emitter (26) is in the form of a light emitting diode emitting light, in particular, in the UV range.

4. The measuring system according to one of the Claims 1 to 3, **characterized in that** the second emitter (30) can be optically excited to emit visible fluorescent light (32).

5. The measuring system according to one of the Claims 1 to 4, **characterized in that** the second emitter (30) is applied as a fluorescent layer to an emission surface of the first emitter (26).

6. The measuring system according to one of the Claims 1 to 5, **characterized in that** the detector (24) includes a photoreceptor (40) for the collective detection of the alternating and the fluorescent light (28, 32).

7. The measuring system according to one of the Claims 1 to 6, **characterized in that** the detector (24) includes two amplifying channels (42, 44) for the wavelength-selective determination of measurement values.

8. The measuring system according to one of the Claims 1 to 7, **characterized in that** the detector (24) includes a lock-in amplifier (46) that can be modulated with the pulse frequency of the alternating light (28) for detecting an alternating light signal component generated by the alternating light (28).

9. The measuring system according to one of the Claims 1 to 8, **characterized in that** the detector (24) comprises an amplifier that functions, in particular, as an integrator (48) for detecting a composite signal generated by the alternating light (28) and the fluorescent light (32).

10. The measuring system according to one of the Claims 1 to 9, **characterized in that** the detector (24) includes a signal processor (50) for determining the signal components of the alternating light and/or the fluorescent light, in particular for subtracting the alternating light signal component from the composite signal.

11. The measuring system according to one of the Claims 1 to 10, **characterized in that** the measuring unit (16) is integrated in a hand-held device (12) and the test element (14) is designed as a disposable for single use in the hand-held device (12).

12. The measuring system according to one of the Claims 1 to 11, **characterized in that** the test element (14) can be coupled to the light source (22) by means of an optical unit (36), in particular a lens or an optical fiber, and that the optical coupling can be monitored by means of a separate signal evaluation in one of the wavelength ranges.

13. A measuring method for determining blood glucose in which a body fluid is applied to a test element (14) that can be used as a disposable element, and the test element (14) is optically scanned by means of a photometric measuring unit (16) comprising a light source (22) and a detector (24), **characterized in that** a first emitter (26) of the light source (22) is actuated to emit pulsed alternating light (28) in a first wavelength range, and that a second emitter (30) formed by a fluorescent substance is excited in a second wavelength range by means of the pulsed first emitter (26) to emit fluorescent light (32) that is superimposed on the alternating light (28), wherein both emitters are jointly oriented towards a measuring surface (34) of the test element (14).

14. The measuring method according to Claim 13, **characterized in that** a signal component related to the alternating light (28) is detected by means of a time-resolved signal detection, preferably by means of a lock-in amplifier (46).

15. The measuring method according to Claim 13 or 14, **characterized in that** in one wavelength range, a measurement value for an analyte in the sample is detected, and **in that**, in the other wavelength range, a control value is detected for the optical coupling of the test element (14) to the measuring unit (16).

## Revendications

1. Système de mesure destiné à la détermination de la glycémie, avec une unité de mesure photométrique (16) qui comprend une source de lumière (22) et un détecteur (24), ainsi qu'un élément de test analytique (14) qui est apte à recevoir un fluide corporel et qui, à des fins de détection optique d'un analyte, peut être mis dans une trajectoire de faisceau (18) entre la source de lumière (22) et le détecteur (24), **caractérisé en ce que** la source de lumière (22) présente un premier émetteur de rayonnement (26) commandé pour émettre de la lumière alternante pulsée (28) dans une première plage de longueurs d'onde, et un deuxième émetteur de rayonnement (30) formé par un luminophore et excité par le premier émetteur de rayonnement pulsé (26) pour émettre de la lumière fluorescente (32) dans une deuxième plage de longueurs d'onde, les deux émetteurs de rayonnement étant orientés ensemble sur une surface de mesure (34) de l'élément de test (14).

2. Système de mesure selon la revendication 1, **caractérisé en ce que** la lumière alternante (28) a une durée d'impulsion et la lumière fluorescente (32) diminue avec une durée de vie fluorescente, et **en ce que** la durée de vie fluorescente est plusieurs fois supérieure à la durée d'impulsion.

3. Système de mesure selon la revendication 1 ou 2, **caractérisé en ce que** le premier émetteur de rayonnement (26) est formé par une diode électroluminescente émettant notamment dans la gamme des UV.

4. Système de mesure selon l'une des revendications 1 à 3, **caractérisé en ce que** le deuxième émetteur de rayonnement (30) est excitable optiquement pour émettre de la lumière fluorescente (32) visible.

5. Système de mesure selon l'une des revendications 1 à 4, **caractérisé en ce que** le deuxième émetteur de rayonnement (30) est appliqué comme couche de luminophore fluorescente sur une surface émettrice du premier émetteur de rayonnement (26).

6. Système de mesure selon l'une des revendications 1 à 5, **caractérisé en ce que** le détecteur (24) comporte un photodétecteur (40) pour la détection commune des lumières alternante et fluorescente (28, 32).

7. Système de mesure selon l'une des revendications 1 à 6, **caractérisé en ce que** le détecteur (24) comporte deux canaux d'amplification (42, 44) pour la détermination sélective en longueur d'onde de la valeur de mesure.

8. Système de mesure selon l'une des revendications 1 à 7, **caractérisé en ce que** le détecteur (24) comporte un amplificateur de verrouillage (46) modulable avec la fréquence d'impulsions de la lumière alternante (28) pour la détection d'une composante de signal de lumière alternante générée par la lumière alternante (28).

9. Système de mesure selon l'une des revendications 1 à 8, **caractérisé en ce que** le détecteur (24) comprend un amplificateur fonctionnant notamment comme intégrateur (48) pour la détection d'un signal de somme généré par la lumière alternante (28) et la lumière fluorescente (32).

10. Système de mesure selon l'une des revendications 1 à 9, **caractérisé en ce que** le détecteur (24) comporte un processeur de signal (50) pour la détermination de composantes de signal de la lumière alternante et/ou lumière fluorescente, en particulier pour la soustraction de la composante de signal de lumière alternante du signal de somme.

11. Système de mesure selon l'une des revendications 1 à 10, **caractérisé en ce que** l'unité de mesure (16) est intégré dans un appareil portatif (12) et l'élément de test (14) est conçu comme consommable destiné à un usage unique dans l'appareil portatif (12).

12. Système de mesure selon l'une des revendications 1 à 11, **caractérisé en ce que** l'élément de test (14) est apte à être couplé à la source de lumière (22) par l'intermédiaire d'une unité optique (36), en particulier une lentille ou un guide de lumière, et **en ce que** le couplage optique est contrôlable par une évaluation de signal séparée dans l'une des plages de longueurs d'onde.

13. Procédé de mesure destiné à la détermination de la glycémie, dans lequel un fluide corporel est déposé sur un élément de test analytique (14) utilisé comme consommable et l'élément de test (14) est exploré optiquement au moyen d'une unité de mesure photométrique (16) comprenant une source de lumière (22) et un détecteur (24), **caractérisé en ce qu**'un premier émetteur de rayonnement (26) de la source de lumière (22) est commandé pour émettre de la lumière alternante pulsée (28) dans une première plage de longueurs d'onde, et en ce qu'un deuxième émetteur de rayonnement (30) formé par un luminophore est excité par le premier émetteur de rayonnement pulsé (26) pour émettre de la lumière fluorescente (32) se superposant à la lumière alternante (28) dans une deuxième plage de longueurs d'onde, les deux émetteurs de rayonnement étant orientés ensemble sur une surface de mesure (34) de l'élément de test (14).

14. Procédé de mesure selon la revendication 13, **caractérisé en ce qu**'une composante de signal associée à la lumière alternante (28) est déterminée par une détection de signal résolue dans le temps de préférence au moyen d'un amplificateur de verrouillage (46).

15. Procédé de mesure selon la revendication 13 ou 14, **caractérisé en ce que** dans l'une des plages de longueurs d'onde, on détecte une valeur de mesure pour un analyte dans l'échantillon, et dans l'autre plage de longueur d'onde on détecte une valeur de contrôle pour le couplage optique de l'élément de test (14) à l'unité de mesure (16).
